(19) Europäisches Patentamt / European Patent Office / Office européen des brevets

(11) EP 3 174 567 B1

(12) EUROPEAN PATENT SPECIFICATION

(45) Date of publication and mention of the grant of the patent: **12.05.2021 Bulletin 2021/19**

(21) Application number: **15759377.3**

(22) Date of filing: **31.07.2015**

(51) Int Cl.: ***A61L 29/08*** *(2006.01)* ***A61L 29/16*** *(2006.01)*

(86) International application number: **PCT/EP2015/001576**

(87) International publication number: **WO 2016/015874 (04.02.2016 Gazette 2016/05)**

# (54) PACLITAXEL-ELUTING BALLOON AND METHOD FOR MANUFACTURING THE SAME

PACLITAXEL-ELUIERENDER BALLON UND VERFAHREN ZUR HERSTELLUNG DAVON

BALLONNET À ÉLUTION DE PACLITAXEL ET SON PROCÉDÉ DE FABRICATION

(84) Designated Contracting States: **AL AT BE BG CH CY CZ DE DK EE ES FI FR GB GR HR HU IE IS IT LI LT LU LV MC MK MT NL NO PL PT RO RS SE SI SK SM TR**

(30) Priority: **01.08.2014 ES 201431178**

(43) Date of publication of application: **07.06.2017 Bulletin 2017/23**

(73) Proprietor: **LVD Biotech S.L.**
**08620 Sant Vicenç dels Horts (ES)**

(72) Inventors:
- **DUOCASTELLA CODINA, Luis**
**E-08620 Sant Vicenç dels Horts (ES)**
- **MOLINA CRISOL, María**
**E-08620 Sant Vicenç dels Horts (ES)**
- **GÓMEZ CASTEL, Alex**
**E-08620 Sant Vicenç dels Horts (ES)**
- **PÉREZ SERRANOS, Isabel**
**E-08620 Sant Vicenç dels Horts (ES)**
- **DURAN PRIU, Marta**
**E-08620 Sant Vicenç dels Horts (ES)**

(74) Representative: **Mohammadian, Dario et al**
**KUKATI**
**Apartado de Correos 34031**
**08080 Barcelona (ES)**

(56) References cited:
**WO-A2-2010/029105**
**US-A1- 2011 295 200**
**US-A1- 2012 095 396**
**US-B1- 6 348 491**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 174 567 B1

## Description

## FIELD OF THE INVENTION

This invention relates to a novel paclitaxel-eluting balloon and a method for manufacturing the same. In particular, it refers to a paclitaxel-eluting balloon with a coating comprising paclitaxel and a lipophilic excipient and to a method for manufacturing the same, wherein the coating shows excellent chemical and mechanical properties to minimize drug losses during balloon catheter management in the patient's body and to improve drug transfer to the tissue to be treated.

## BACKGROUND

Today it is frequent to treat occluded or partially occluded arteries for the purpose of restoring blood flow. This treatment is called angioplasty and is performed both in coronary arteries and in peripheral arteries. In the early days, balloon catheters were used which, once inserted in the patient's body and reached the occlusion to be treated, the balloon was inflated for the purpose of dilating the affected artery. Angioplasty basically relates to the treatment of a stenosis. Stenosis is a narrowing of a body duct, such as a blood vessel, which can cause a severe injury. However, it was observed that treatment with a balloon catheter also caused damage to the treated blood vessel tissue, so that restenosis occurred in the same place due to cell proliferation and neointimal hyperplasia. Therefore, the vessel narrowing occurred again and another treatment with the balloon catheter was required.

To treat stenosis and avoid restenosis stents were designed, which are intravascular prostheses used to keep open a previously stenosed vessel. In most cases it is a mesh or metal coil with a cylindrical or tubular shape. However, although these stents provide very good results in reducing the incidence of restenosis, they have the problem that they do not reduce intimal hyperplasia and also entail a hyperproliferative behavior. With the stents, it has also been observed the problem of marked slowing down and impairment of vascular repair, which can be clinically evidenced as the occurrence of stent thrombosis and development of neoatherosclerosis. Other problems related to the stents with a polymer coating can be persistent inflammation phenomena, hypersensitivity and deficient vascular repair.

A further progress in the development of stents was the drug-eluting stents, that prevent restenosis in the stent choosing a drug for the treatment of neointimal proliferation. In this case the stent is coated with a polymer containing an adequate drug. However, there is still the problem of the possible occurrence of thrombosis that has not been solved with drug-eluting stents. In addition, the polymer coating of the stent has caused other problems. When implanting the drug-eluting stent, persistent inflammation phenomena, hypersensitivity and deficient vascular repair are often observed.

Therefore, in recent years a possible alternative to drug-eluting stents has been developed. These are the so-called drug-eluting balloons, in particular paclitaxel-eluting balloons. It is a balloon catheter wherein the balloon is coated with a composition containing paclitaxel. These drug-eluting balloon catheters can be used both in coronary angioplasty and in peripheral angioplasty to dilate a blood vessel affected by an obstruction or narrowing. To prevent a new occlusion due to restenosis, the balloon is coated with an antiproliferative drug, for instance paclitaxel, which inhibits cell proliferation of the smooth muscle cells and neointimal hyperplasia and blocks these muscle cells throughout the inflammation period, which usually lasts a few days.

The use of a drug-eluting balloon catheter comprises in general three stages. The first stage is navigation of the balloon catheter from the insertion point in the patient to the lesion to be treated. During this navigation, the coating is exposed to blood flow and there is a risk that it is detached and that, thus, the drug is lost before reaching the lesion to be treated. Therefore, the drug effect will be reduced or even removed and there can be the problem that restenosis is not avoided. The second stage is drug transfer to the arterial wall once the balloon catheter has reached the lesion to be treated. The coating must have a high capacity to transfer the drug or at least not block its transfer to the artery. The known coatings have the problem of a reduced transfer of the drug or a reduced time of residence of the drug in the artery, which leads to a higher risk of restenosis. Drug transfer in the second stage occurs mechanically when the coated balloon is expanded and contacts the arterial wall. The pressure of the coated balloon on the arterial wall causes the transfer of the drug to the artery. The transfer time is usually from half a minute to one minute in the case of coronary angioplasty and it can be a few minutes in the case of peripheral angioplasty. The balloon is subsequently deflated for removal. On the one hand, there can be a problem that not all the drug is transferred during the transfer and some still remains in the balloon coating. On the other hand, there is also the problem that the artery has not absorbed all the drug transferred and that a part remains attached to the tissue. Then, when the balloon is deflated, the blood stream flows again through the artery and due to its strength can carry away downstream significant amounts of the non-absorbed drug attached to the tissue or the remaining coating particles in the balloon catheter, so that the drug carried away cannot act in the lesion to be treated. Therefore, there is a greater risk of restenosis.

In particular, today's paclitaxel-eluting balloons still have the problem that the results in the angioplasty vary markedly. The problems result both from how the balloon is coated with paclitaxel and of the composition the coating and/or its structure. For instance, a poor application of the coating has the problem of a low adhesion of the coating to

the balloon. Consequently, delaminations of the coating are seen during balloon handling, which increases the risk of losses during navigation. There is also a problem that a poor application forms micropores in the coating. Then, once the balloon is inserted in the body of the patient, the blood flow can enter these micropores, which allows to even reach the balloon surface. This causes a detachment of the coating causing a high partial or almost complete loss of the drug, so the treatment will not be successful.

Therefore, there is a need to provide a paclitaxel-eluting balloon with a coating that adheres well to the balloon, allowing a navigation without paclitaxel losses and at the same time having a high drug transfer capacity in order to transfer all paclitaxel during contact between the balloon and the artery. There is also a need for a coating for a paclitaxel-eluting balloon which allows a high degree of drug absorption to prevent losses during balloon removal. WO2010/029105 A2 discloses fatty acid triglyceride mixtures for the controlled delivery of bioactive substances, in particular to the vascular wall and/or biocompatible coating of medical devices, e.g. an expandable stent.

The object of this invention is to provide a paclitaxel-eluting balloon that shows a better performance with respect to both the navigation stage and the transfer stage. In particular, the object is to provide a coating for a paclitaxel-eluting balloon which allows simultaneously 1) a good adhesion to the balloon, 2) a good drug retention when in contact with the blood flow, and 3) a good drug transfer to the tissue to be treated so that the drug has both a short- and long-term effect in order to minimize and eliminate the risk of restenosis.

## SUMMARY OF THE INVENTION

Some or all of the problems of the state of the art described above are solved with a balloon catheter coating composition according to independent claim 1 and a paclitaxel-eluting balloon catheter according to independent claim 8. The preferred embodiments are described in the dependent claims.

The paclitaxel-eluting balloon according to the invention presents a coating comprising paclitaxel and a lipophilic excipient.

The invention also relates to a method for manufacturing a paclitaxel-eluting balloon comprising the stages of providing a balloon catheter and applying a formulation comprising paclitaxel, at least one lipophilic excipient and a solvent.

The lipophilic excipient is selected from the group comprising a triglyceride according to formula 1, a compound according to formula 2 or a mixture thereof, wherein the compound of formula 1 is as follows:

(Formula 1)

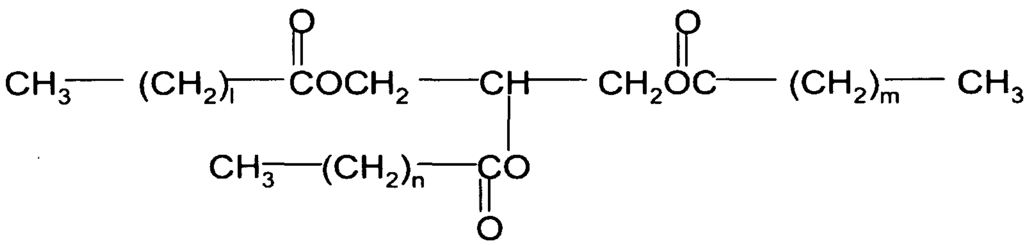

wherein l, m and n are independently an integer from 4 to 12; and
wherein the compound of formula 2 is as follows:

(Formula 2)

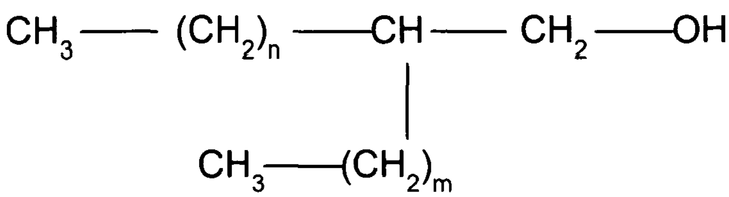

wherein n is an integer from 5 to 11 and m is an integer from 5 to 7.

## FIGURES

FIG. 1 schematically illustrates a balloon catheter by way of example which can be used in the present invention.
FIGs. 2a and 2b are SEM images at different resolution of a coating according to the invention comprising paclitaxel in crystalline form.

## DETAILED DESCRIPTION OF THE INVENTION

It has been found that a composition of paclitaxel with a sufficiently lipophilic excipient can solve some or all problems of the state of the art. This composition applied as coating to a balloon catheter can retain the drug paclitaxel in the coating effectively. In addition, it is observed a fast drug transfer to the tissue to be treated combined with a long-lasting therapeutic effect.

In particular, the composition for the coating of a balloon catheter comprises paclitaxel and an excipient selected from the group comprising a triglyceride according to formula 1, a compound according to formula 2 or a mixture thereof, wherein the compound of formula 1 is as follows:

(Formula 1)

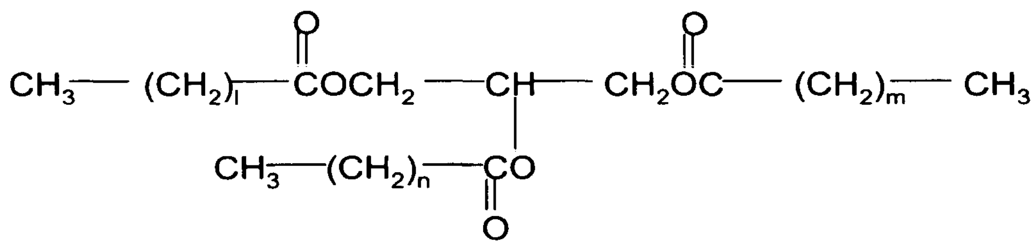

wherein l, m and n are independently an integer from 4 to 12; and
wherein the compound of formula 2 is as follows:

(Formula 2)

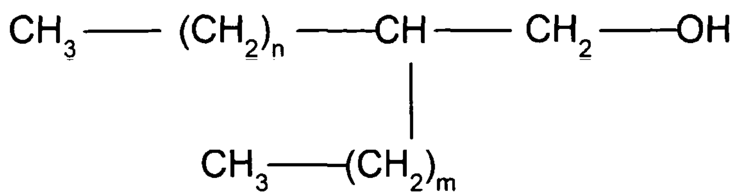

wherein n is an integer from 5 to 11 and m is an integer from 5 to 7, and wherein the paclitaxel/excipient ratio in weight ranges from 60/40 to 90/10.

This composition is surprisingly effective to retain paclitaxel during the balloon catheter navigation stage so that the blood flow cannot extract it. Furthermore, this composition enables a fast, effective transfer, with a good absorption by the tissue to be treated so that paclitaxel losses during the treatment are minimized and the maximum amount can be administered. Therefore, the risk of restenosis can be minimized.

Fig. 1 shows a balloon catheter 100 by way of example. However, the invention is not limited to this balloon catheter, but also considers all types of balloon catheters. The balloon catheter 100 comprises a catheter body 102 with a proximal end 104 and a distal end 106. In the proximal end 104 a luer connector 108 can be provided to attach various accessories and an inlet 110 which allows balloon inflation. In the other side, in the distal end 106 the balloon 112, also called inflatable segment, is located, which ends in a soft, non-invasive tip 114. In addition, the balloon catheter 100 can comprise radiopaque markers. The catheter body 102 can have for instance a proximal simple lumen and distal coaxial lumen configuration (not shown). For instance, the simple lumen can be constituted by a hypotube (not shown) which allows the passage of the inflation liquid from the inlet 110 to the balloon 112, while the coaxial lumen can comprise an inner lumen of wire guide passage and an external inflation lumen in direct connection with the hypotube.

This balloon catheter can be used as drug-eluting balloon catheter applying to the balloon a coating containing the drug to be eluted. The balloon coating according to the present invention has a composition comprising paclitaxel and an excipient selected from the group comprising a triglyceride according to formula 1, a compound according to formula 2, or a mixture thereof. In some aspects of this invention, the balloon coating consists of paclitaxel and an excipient selected from the group comprising a triglyceride according to formula 1, a compound according to formula 2, or a mixture thereof.

The combination of paclitaxel with a triglyceride according to formula 1, a compound according to formula 2 or a mixture thereof provides a balloon catheter with good properties in both the navigation stage and in the transfer stage, as mentioned above. In addition, the coating shows a high safety for the patient because the excipients according to the invention are considered non-toxic and safe for humans. The excipients according to the invention improve the integrity and homogeneity of the coating and reduce and even remove the presence of micropores in the coating, with the result that less paclitaxel is lost during navigation of the balloon catheter to the lesion to be treated. In addition, it has the surprising effect that they improve simultaneously transfer and absorption of paclitaxel in the tissue to be treated. It has been observed that during balloon expansion the exposure is increased and paclitaxel release and transfer to the tissue to be treated speed up, which allows to reach the necessary therapeutic levels of paclitaxel. The tissue to be treated can be any blood vessel wall where there is a lesion, for instance an arterial wall.

In particular, it is thought that the hydrocarbon chains of the excipients create an environment which is lipophilic enough for paclitaxel, that, due to its molecular structure is also lipophilic, is dissolved in the excipient. In addition, due to this composition, the coating is also lipophilic, so that detachments thereof into the blood flow or other hydrophilic environments can also be prevented. Therefore, the blood flow contacting the coating during navigation cannot dissolve or extract paclitaxel and, thus, losses are very low. Furthermore, the lipophilic nature of the excipients according to the invention allows to better interact with the tissue to be treated during the transfer and thus facilitate paclitaxel absorption. Therefore, the excipients according to the invention show a synergistic effect which allows to obtain a higher effect of paclitaxel on the treatment.

The drug to excipient ratio is important to control the coating properties. In this invention, a drug/excipient ratio in weight that ranges from 60/40 to 90/10 is used. Preferably, the drug/excipient ratio in weight ranges from 70/30 to 90/10, more preferably from 70/30 to 85/15 and even more preferably from 75/25 to 85/15. If the drug/excipient ratio in weight of the coating is within these ranges, a good balance of properties of integrity and drug losses is obtained. However, an excellent balance between a very low loss of the drug during navigation and a high drug transfer to the tissue to be treated is achieved with a drug/excipient ratio in weight from 83/17 to 78/22, preferably with a drug/excipient ratio in weight of about 80/20. Conversely, if a drug/excipient ratio in weight of over 90/10 is used, the risk of drug is loss increases during navigation, as the amount of excipient is not enough to ensure the integrity properties and losses in navigation. On the other hand, if a drug/excipient ratio in weight of less than 60/40 is used, drug transfer to the tissue to be treated can be made difficult and even blocked. In addition, a very thick coating would be needed to be able to apply the necessary dose of paclitaxel (as explained below), which would increase further the profile of the balloon catheter preventing access to the narrowest blood vessels.

The paclitaxel used in the coating is included at an amount that allows to obtain a high efficacy of the treatment. The amount is usually expressed as dose per area of the expanded balloon. The coating of this invention can comprise a dose of paclitaxel from 1.5 $\mu g/mm^2$ to 5.0 $\mu g/mm^2$. Preferably, the dose ranges from 2.0 $\mu g/mm^2$ to 4.0 $\mu g/mm^2$ and more preferably from 2.5 $\mu g/mm^2$ to 3.5 $\mu g/mm^2$. In particular, the dose can be 2.5, 2.6, 2.7, 2.8, 2.9, 3.0, 3.1, 3.2, 3.3, 3.4 or 3.5 $\mu g/mm^2$ or a range of any combination thereof. With these doses a high treatment efficacy is obtained and a possible restenosis is significantly reduced in the lesion to be treated.

Besides the dose of paclitaxel, it is also important how paclitaxel is present in the composition and, thus, in the coating. Paclitaxel can exist both in amorphous and in crystalline form. The crystalline form can be also anhydrous or hydrate. In general, the form of paclitaxel in the coating is controlled by the dissolvent used in the preparation of the formulation for the coating. Paclitaxel form can be also controlled by the coating application technology. This will be described in further detail in relation to the method describing how a balloon catheter is coated according to the present invention.

Both paclitaxel forms have different properties and the result of the treatment depends largely on which form is used. For instance, the amorphous form allows to obtain a highly uniform coating, and its appearance is a film protecting the coating from losses in the navigation stage and a good transfer of the drug to the arterial wall. However, drug retention in the tissue is short over time and its effect decreases fast. This is because the amorphous form is fast diffused in the blood vessels and is also dissolved fast in blood and, thus, disappears from the tissue to be treated in a short time. Usually at 24 hours following administration only about 1 % of the drug remains. Conversely, paclitaxel in crystalline form produces a coating with a lower adhesion than amorphous paclitaxel, which can cause more losses during the navigation stage. Therefore, it is recommended to use crystalline paclitaxel with a small crystal size because this can minimize the risk of losses. Preferably, the crystal size is the range of nanometers. However, crystalline paclitaxel has a high transfer capacity of the drug to the tissue to be treated and shows an excellent drug retention in the tissue to be treated over time, so that a therapeutic effect is obtained that can last up to 28 days after drug administration. This is due to the crystalinity and its lipophilic nature, as it is solubilized only slowly, allowing a dosing sustained over time. Therefore, crystalline paclitaxel shows a high biological effectiveness. Consequently, in this invention paclitaxel is preferably used in crystalline form, that can be anhydrous, hydrate or a mixture thereof, as the inflammation in the lesion to be treated can last a few days and a sustained dosing of longer effect allows to obtain better results in the treatment with less probable restenosis.

FIGs. 2a and 2b are SEM images at different resolution that show a coating according to the invention comprising paclitaxel in crystalline form. These images were taken with a scanning electron microscope (SEM) XL 30, FEI. A uniform coating can be seen, wherein crystalline paclitaxel is coated by the excipient. Therefore, paclitaxel is protected during application of the coated balloon catheter in the body of a patient. In the case of FIGs. 2a and 2b, the excipient is a compound according to formula 1.

In some aspects of this invention a mixture of paclitaxel in crystalline and in amorphous form can be also used. A combination of both paclitaxel forms has the surprising effect that the results obtained in the treatment are better as compared to coatings containing a single paclitaxel form. It is believed that this is due to the combination of one part in amorphous form and another part in crystalline form, because it shows a synergistic effect for the immediate effect of amorphous paclitaxel and for the long lasting effect of crystalline paclitaxel. In addition, the amorphous form helps improve adhesion of the coating to the balloon, minimizing further drug losses during the navigation stage. However, it is necessary that the part of crystalline paclitaxel is at least 50 % in weight to obtain a long-lasting effect of paclitaxel. Less than 50 % in weight of crystalline paclitaxel can cause a reduced, less effective long-lasting effect, so that it can increase the risk of restenosis. Preferably, the amorphous/crystalline paclitaxel ratio in weight is 10/90 to 50/50, more preferably from 10/90 to 40/60 and even more preferably from 10/90 to 30/70.

In this invention a combination of paclitaxel and excipient is used, because a coating only consisting of paclitaxel would have significant losses during the navigation and a reduced transfer to the tissue to be treated. The excipients in the coating according to this invention are selected from the group comprising a triglyceride according to formula 1, a compound according to formula 2, or a mixture thereof. In some aspects of the invention, a triglyceride according to formula 1 is used as excipient. In other aspects of the invention a compound according to formula 2 is used as excipient. And in other aspects of the invention, a combination of a triglyceride according to formula 1 with a compound according to formula 2 is used as excipient. These excipients offer an excellent interaction with paclitaxel due to their lipophilic nature, resulting in a homogeneous coating with an excellent integrity without risk that delaminations of the coating occur during navigation or that micropores are formed in the coating.

With these excipients, the following effect is observed in particular with the compound according to formula 2. When the balloon catheter contacts the blood flow, a slight solubilization of the coating or wetting effect is produced, which leads to a gel appearance. In this report the terms "gel effect" or "coating of gel type" can also be used for it. The gel type coating minimizes and can even remove the possibility of detachment of particles and improves drug transfer through the tissue. It is thought that the excipient according to the invention promotes the formation of the gel effect reducing navigation losses and enhancing transfer to the tissue. With the compound according to formula 2, the gel effect is greater due to its amphiphilic nature.

Preferably, these excipients are liquid at room temperature, i.e., at a temperature ranging from 20 to 25 °C, which improves even further homogeneity, integrity and resistance to manipulation of the coating.

To obtain a good behavior in terms of navigation losses excipients according to the invention can also be used, that are solid at room temperature. However, these solid excipients show the disadvantage that the coatings are dry, show a low resistance to manipulation and require great caution when handling the balloon catheter.

A lipophilic excipient that can be used according to the invention is a triglyceride according to formula 1:

(Formula 1)

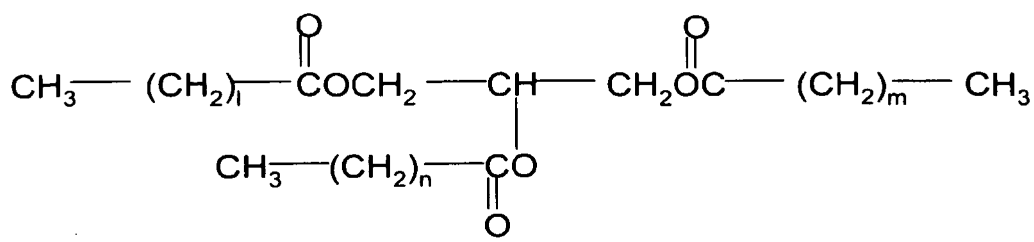

wherein l, m and n are independently an integer from 4 to 12. Preferably, l, m and n are independently an integer from 6 to 10, even more preferably of 6 to 8. In one aspect of this invention, l, m and n can be the same integer, so that the three groups of carbonic acid are equal. In another aspect of this invention, l and m can be the same integer, while n is a different number, so that it is a mixed triglyceride of two different carbonic acids. And in another aspect of this invention, l, m and n are each a different integer, so that it is a mixed triglyceride of three different carbonic acids.

Preferably, the triglyceride according to formula 1 is a triglyceride in that at least one of l, m or n is 6, even more preferably two of l, m or n are 6, and even more preferably l = m = n = 6. Therefore, at least one of the three groups of carbonic acid is caprylic acid, preferably two of the three groups of carbonic acid are caprylic acid, and even more preferably all the three groups of carbonic acid are caprylic acid. These triglycerides give an excellent balance of properties on coating, in terms of reduced drug loss during navigation and an excellent transfer thereof to the tissue to be treated.

Preferably the triglyceride of caprylic acid is used.

In another aspect of this invention, the coating can comprise two or more different triglycerides according to formula 1. This can allow the adjustment of some coating properties. However, at least one of these triglycerides has a high caprylic acid content, preferably it is a caprylic acid triglyceride, so that more than 50 % in weight of the carbonic acid groups of triglycerides in total is caprylic acid.

Another lipophilic excipient that can be used according to the invention is a compound according to formula 2:

(Formula 2)

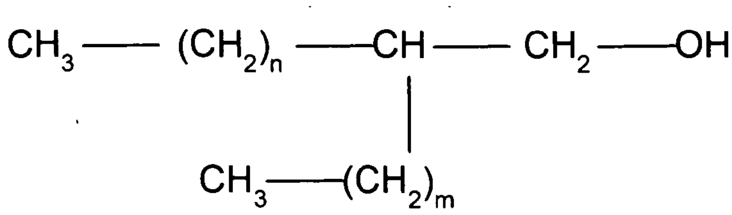

wherein n is an integer from 5 to 11 and m is an integer from 5 to 9. In some aspects of this invention, the compound according to formula 2 is a compound where n ranges from 7 to 11, preferably from 9 to 11. In other aspects of the invention, the compound according to formula 2 is a compound wherein m ranges from 5 to 9, preferably from 7 to 9. In some preferred aspects of this invention n is 9, while m ranges from 5 to 9, preferably from 7 to 9. In other preferred aspects of this invention m is 7, while n ranges from 5 to 11, preferably from 7 to 11, more preferably from 7 to 9. Preferably the compounds according to formula 2 which are liquid at room temperature are used. In the most preferable aspect of this invention, it is a compound of formula 2 called 2-octyldodecanol wherein n is 9 and m is 7. With this compound a coating is obtained with an excellent balance of properties for navigation and drug transfer.

In some aspects of this invention the coating comprises a combination of a triglyceride according to formula 1 and a compound according to formula 2. The weight ratio of triglyceride according to formula 1 and the compound according to formula 2 ranges from 1/99 to 99/1, preferably from 10/90 to 90/10, even more preferably from 20/80 to 80/20. Preferably the combination is a mixture of the triglyceride of caprylic acid and 2-octyldodecanol. The combination of these two excipients allows to better modulate the properties of the coating and the gel effect in the navigation stage.

The paclitaxel-eluting balloon according to this invention is manufactured by the method described below. The method comprises the steps of providing a balloon catheter and coating partially or completely the outer surface of the balloon with a composition comprising paclitaxel and an excipient selected from the group comprising a triglyceride according to formula 1, a compound according to formula 2 or a mixture thereof. In one aspect of the invention, the step of coating the balloon is performed with a composition consisting of paclitaxel and an excipient selected from the group comprising a triglyceride according to formula 1, a compound according to formula 2 or a mixture thereof.

The step of providing the balloon comprises providing a balloon catheter wherein the balloon is in folded state or in inflated state. The step of coating the balloon is then performed with the balloon in folded state or in inflated state. However, paclitaxel dose in the coating will be the same, regardless of whether the balloon was coated in folded or inflated state.

The balloon coating step can be performed using an application by immersion, by micropipette or by spray. Therefore, the balloon can be coated by immersion it in a formulation comprising the composition to be coated, applying to it this formulation by a micropipette or spraying this formulation over it. For the purposes of this invention, the term "spray" can also mean "pulverization".

Preferably spray application is used. This application has the advantage of obtaining a coating with an excellent adhesion to the balloon, durability and mechanical properties. Therefore, coating losses during handling and navigation are reduced. In addition, the spray technique allows to obtain a coating wherein the drug is distributed homogeneously in the coating, so that throughout the length of the coated balloon the same dose is found. This makes no small focuses of restenosis are caused in the lesion to be treated because along the area to be treated paclitaxel is transferred in a sufficient dose. In addition, the spray technique allows a high repeatability in the dose of the drug. It has been also observed that the coating applied by spray provides good biological properties, such as very fast absorption in at least 30 seconds.

The coating obtained by spray is uniform, homogeneous and highly resistant due to the good adhesion of the composition of the coating to the balloon material. The good adhesion also makes that only a slight solubilization of the coating is observed during navigation, the so-called gel effect, i.e., losses during navigation are very low, and excellent drug transfer to the arterial wall is obtained.

With the spray technique even the coating structure can be controlled. A single-layer coating or a multi-layer coating can be obtained. A single-layer is obtained using a formulation with a high concentration in paclitaxel to obtain

the desired dose. Then a single run is made spraying the formulation over the balloon. However, a multilayer is obtained using a formulation with a lower paclitaxel concentration and spraying the formulation in several runs on the balloon until the desired dose is obtained, that can range from 1.5 to 5.0 $\mu g/mm^2$ as described above.

The advantage of a multi-layer coating versus a single-layer coating is the integrity of the coating in view of the handling and navigation. In addition, coating adhesion to the balloon is better in the case of a multilayer coating, which reduces delaminations during handling and prevents formation of micropores in the coating. Therefore, the multi-layer coating will have fewer losses during navigation than the single-layer coating.

The formulation for coating the balloon comprises the composition comprising paclitaxel and an excipient selected from the group comprising a triglyceride according to formula 1, a compound according to formula 2 or a mixture thereof. For this, the drug and the excipient are dissolved in a solvent or in a mixture of solvents at a concentration from 1 to 10 % non-volatiles, preferably from 2 to 8 % non-volatiles, more preferably from 3 to 6 % non-volatiles, even more preferably from 4 to 5 % non-volatiles. In particular, the concentration can be 4.0, 4.1, 4.2, 4.3, 4.4, 4.5, 4.6, 4.7, 4.8, 4.9 or 5.0 % non-volatiles or a range of any combination thereof. This liquid formulation is then applied to the balloon spraying it over the same and the balloon is subsequently dried.

The solvent for preparing the formulation plays a major role for paclitaxel. Based on the solvent used, paclitaxel is obtained in amorphous or crystalline form. Amorphous paclitaxel is obtained with apolar organic solvents in the absence of water. Examples of apolar organic solvents comprise tetrahydrofuran, acetone, chloroform or dichloromethane. Crystalline paclitaxel in its anhydrous form is obtained with polar organic solvents in the absence of water, that is, with dry polar organic solvents. Examples of polar organic solvents comprise methanol, ethanol or isopropanol. Crystalline paclitaxel in its hydrate form is obtained with organic solvents that are water-soluble and contain water. The organic solvent/water ratio in volume can range from 99/1 to 80/20, preferably from 98/2 to 85/15, even more preferably from 97/3 to 90/10. Specific examples are 96 % ethanol in weight, tetrahydrofuran/water at a 9:1 ratio in volume or acetone/water at a 9/1 ratio in volume. Preferably, ethanol is used as solvent for the formulation that can be dry ethanol or 96 % ethanol in weight.

From the same formulation a coating comprising paclitaxel in crystalline and in amorphous form can be even obtained. For this, an organic solvent is used that can provide crystalline paclitaxel as one of those described above and, based on the drying conditions used, obtaining paclitaxel in crystalline or amorphous state can be promoted. For instance fast drying usually results in more amorphous than crystalline paclitaxel.

Once the balloon has been coated, it is allowed to dry until all the solvent used to prepare the formulation has been removed. A sterilization stage is then performed, the balloon catheter is packaged and stored until it is needed for a patient.

The balloon catheter coated according to this invention with a composition comprising paclitaxel and an excipient selected from the group comprise a triglyceride according to formula 1, a compound according to formula 2 or a mixture thereof can be used in treatments and for the prevention of restenosis by administration of the antiproliferative drug. The balloon catheter can be used both in coronary angioplasty and in peripheral angioplasty. The balloon catheter is inserted folded in a patient needing the appropriate treatment, the balloon is navigated to the lesion to be treated and then the balloon is dilated to reach the diameter of the blood vessel to be treated. Balloon dilation can last about 30 to 40 seconds in the case of coronary angioplasty and some minutes in peripheral angioplasty, time in which drug transfer from the coating to the tissue to be treated. After drug administration, the balloon is fully deflated and removed from the patient.

The balloon catheter according to the invention has few drug losses during navigation due to the excellent properties of the coating and its composition and shows an excellent drug transfer to the tissue to be treated so that the results obtained in the treatment are very good.

The invention will be detailed by way of example in the following experimental examples.

What has been described comprises several embodiments by way of example. It is understood that the person of ordinary skill in the art would conclude different permutations and possible combinations of the various embodiments and aspects described after a direct, objective reading of this disclosure. Therefore, the embodiments and main aspects have been described, understanding that they comprise the other combinations, variations and modifications, provided they are comprised in the scope of protection defined by the claims. The person of ordinary skill in the art would understand that the description of the embodiments given does not limit the invention and the drawings do not either.

## Examples

Preparation of a formulation 1 with the excipient tricaprylin

Formulation 1 was prepared dissolving 80 mg of paclitaxel and 20 mg of tricaprylin in 2.75 ml of $THF/H_2O$ 9:1.

Preparation of a formulation 2 with the excipient 2-octvldodecanol

Formulation 2 was prepared dissolving 80 mg of paclitaxel and 20 mg of 2-octyldodecanol in 2.75 ml of THF/$H_2O$ 9:1.

Process of coating a balloon catheter

9 PTCA balloon catheters (PCTA: "Percutaneous transluminal coronary angioplasty") (3.0 mm in diameter and 20 mm in length, Xperience manufactured by LVD Biotech) were inflated between 1-3 atm. The inflated balloons were coated with formulation 1 by a spray technique. The expected amount of drug was obtained on the balloon (3 $\mu$g/mm$^2$). After folding the balloons, these were sterilized for animal test.

9 PTCA balloon catheters (3.0 mm in diameter and 20 mm in length, Xperience manufactured by LVD Biotech) were inflated between 1-3 atm. The inflated balloons were coated with formulation 2 by a spray technique. The expected amount of drug was obtained on the balloon (3 $\mu$g/mm$^2$). After folding the balloons, these were sterilized for animal tests.

9 PTCA balloon catheters (3.0 mm in diameter and 20 mm in length, Xperience manufactured by LVD Biotech) were folded with three wings under vacuum. The folded balloons were coated by spray with formulation 1. The expected amount of drug was obtained on the balloon (3 $\mu$g/mm$^2$). Finally, the coated balloon catheters were sterilized for animal test.

Application of the balloon catheter in the animal model

For carrying out the experimental study, in the application of a balloon catheter 14 domestic pigs and 42 PTCA balloon catheters (3.0 mm in diameter and 20 mm in length) were employed, whose randomization was as follows:

- 18 PTCA balloon catheters coated with the formulation 1 (Xperience, manufactured by LVD Biotech)
- 9 PTCA balloon catheters coated with the formulation 2 (Xperience, manufactured by LVD Biotech)
- 7 control PTCA balloon catheters coated with a mixture of paclitaxel and Iopromide (Sequent Please, manufactured by B.Braun)
- 8 uncoated control PTCA balloon catheters (Xperience, manufactured by LVD Biotech)

For the purpose of implanting the devices with a stent:artery size ratio of 1.1 - 1.2, the best segment was located in each of the three coronary arteries (LAD, LCX and RCA). The inflation pressure was adjusted to reach the desired overdimension. After implanting the stents (Architect, CoCr stent manufactured by LVD Biotech), post-dilation was performed with the different balloons of the same caliber as the stent implanted. Drug release into the target segment was performed over 60 seconds.

Angiographic analysis: after finishing the described method in each artery, a new coronary angiography was performed after intracoronary administration of nitroglycerin to measure the minimum luminal diameter of the stent. At 28 days, a control coronary angiography was performed for measuring the minimum luminal diameter at follow-up. The angiographic restenosis parameters are calculated:

Late loss = minimum initial luminal diameter – minimum luminal diameter follow-up

Percentage of stenosis by angiography = [1 – (minimum luminal diameter follow-up /reference diameter)] x 100.

| | Tricaprylin | 2-Octyldodecanol | Sequent Please | Control |
|---|---|---|---|---|
| Late loss (mm) | 0.61 | 0.81 | 1.12 | 1.41 |
| Angiographic stenosis (%) | 12.58 | 15.50 | 17.11 | 31.56 |

## Claims

1. A balloon catheter coating composition comprising paclitaxel and an excipient selected from the group comprising at least a triglyceride according to formula 1, at least a compound according to formula 2 or a mixture thereof, wherein the compound of formula 1 is as follows:

(Formula 1)

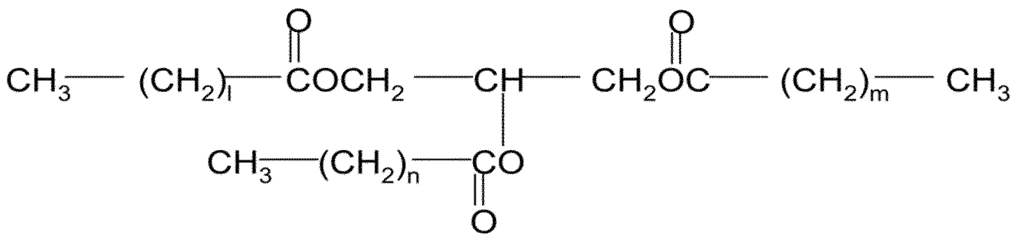

wherein l, m and n are independently an integer from 4 to 12; and
wherein the compound of formula 2 is as follows:

(Formula 2)

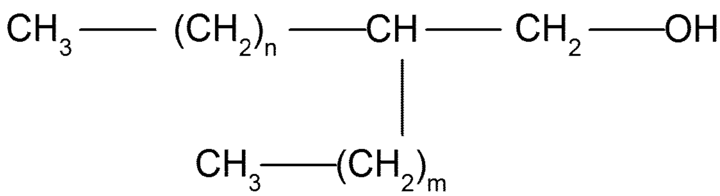

wherein n is an integer from 5 to 11 and m is an integer from 5 to 7; and
wherein the paclitaxel/excipient ratio in weight ranges from 60/40 to 90/10.

2. The composition according to claim 1, wherein paclitaxel is present in amorphous and crystalline form wherein the weight ratio of amorphous/crystalline is from 10/90 to 50/50.

3. The composition according to claim 1, wherein the excipient is a liquid at a temperature from 20 to 25 °C.

4. The composition according to claim 1, wherein the compound according to formula 1 is a mixed triglyceride with three different carbonic acids, of two different carbonic acids or a triglyceride wherein l = m = n.

5. The composition according to claim 4, wherein the triglyceride according to formula 1 is caprylic acid triglyceride with l = m = n = 6.

6. The composition according to claim 1,
wherein the compound according to formula 2 is a compound wherein n = 9 and m = 5 a 9;
or wherein the compound according to formula 2 is a compound wherein n = 5 a 11 and m = 7;
or wherein the compound according to formula 2 is 2-octyldodecanol.

7. The composition according to claim 1, wherein the composition comprises paclitaxel, a triglyceride according to formula 1 and a compound according to formula 2, wherein the weight ratio of triglyceride according to formula 1 / compound according to formula 2 is from 1/99 to 99/1.

8. Paclitaxel-eluting balloon catheter, whose balloon is partially or completely coated, **characterized in that** the coating comprises the composition according to claim 1, wherein the excipient is selected from the group comprising two or more different triglycerides according to formula 1, wherein for at least one of these triglycerides more than 50% in weight of the carbonic acid groups of the at least one triglyceride in total is caprylic acid.

**9.** Paclitaxel-eluting balloon catheter according to claim 8, wherein the dose of paclitaxel in the coating ranges from 1.5 $\mu g/mm^2$ to 5 $\mu g/mm^2$.

**10.** Method for manufacturing a paclitaxel-eluting balloon catheter comprising the following steps:

providing a balloon catheter; and
coating partially or completely the balloon catheter with a composition according to claim 1.

**11.** Method according to claim 10, wherein coating the balloon comprises the steps of:

providing a formulation of the composition according to claim 1 in a solvent;
spraying the formulation at least once over the balloon in order to coat partially or completely its outer surface; and drying the coating.

**12.** Method according to claim 11, wherein the formulation has a concentration between 1 to 10 % non-volatiles.

**13.** Method according to claim 11, wherein the solvent is a polar organic solvent or a water-soluble organic solvent containing water in a volume ratio of solvent/water from 99/1 to 80/20, wherein the solvent is selected from the group comprising dry ethanol, dry methanol, dry isopropanol, ethanol/water 96/4 (w/w), THF/water 9/1 (v/v) or acetone/water 9/1 (v/v).

**14.** Method according to claim 11, wherein the formulation is sprayed at least two, at least three or more than three times over the balloon to obtain a multilayer coating.

**15.** Use of the composition according to claim 1 for preparing a device for the treatment of coronary or peripheral angioplasty.

**Patentansprüche**

**1.** Beschichtungszusammensetzung für Ballonkatheter, umfassend Paclitaxel und einen Hilfsstoff, ausgewählt aus der Gruppe, umfassend mindestens ein Triglycerid gemäß Formel 1, mindestens eine Verbindung gemäß Formel 2 oder eine Mischung davon, wobei die Verbindung der Formel 1 wie folgt ist:

(Formel 1)

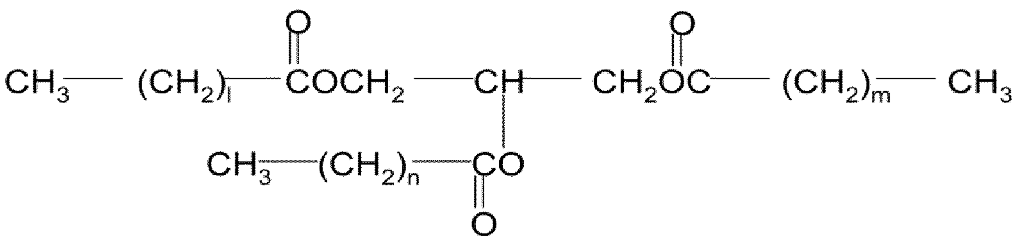

wobei l, m und n unabhängig voneinander eine ganze Zahl von 4 bis 12 sind; und
wobei die Verbindung der Formel 2 wie folgt ist:

(Formel 2)

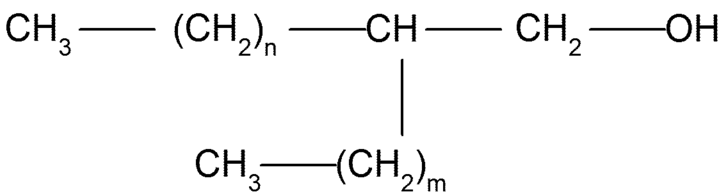

wobei n eine ganze Zahl von 5 bis 11 ist und m eine ganze Zahl von 5 bis 7 ist; und
wobei das Paclitaxel/Hilfsstoff-Gewichtsverhältnis im Bereich von 60/40 bis 90/10 liegt.

**2.** Zusammensetzung gemäß Anspruch 1, wobei Paclitaxel in amorpher und kristalliner Form vorliegt, wobei das Gewichtsverhältnis von amorph/kristallin von 10/90 bis 50/50 ist.

**3.** Zusammensetzung nach Anspruch 1, wobei der Hilfsstoff eine Flüssigkeit bei einer Temperatur von 20 bis 25 °C ist.

**4.** Zusammensetzung gemäß Anspruch 1, wobei die Verbindung gemäß Formel 1 ein gemischtes Triglycerid mit drei verschiedenen Kohlensäuren, zwei verschiedenen Kohlensäuren oder ein Triglycerid mit l = m = n ist.

**5.** Zusammensetzung nach Anspruch 4, wobei das Triglycerid gemäß Formel 1 Caprylsäuretriglycerid mit l = m = n = 6 ist.

**6.** Zusammensetzung gemäß Anspruch 1,
wobei die Verbindung gemäß Formel 2 eine Verbindung ist, wobei n = 9 und m = 5 a 9 ist;
oder wobei die Verbindung gemäß Formel 2 eine Verbindung ist, wobei n = 5 a 11 und m = 7 ist;
oder wobei die Verbindung gemäß Formel 2 2-Octyldodecanol ist.

**7.** Zusammensetzung gemäß Anspruch 1, wobei die Zusammensetzung Paclitaxel, ein Triglycerid gemäß Formel 1 und eine Verbindung gemäß Formel 2 umfasst, wobei das Gewichtsverhältnis von Triglycerid gemäß Formel 1/ Verbindung nach Formel 2 von 1/99 bis 99/1 ist.

**8.** Paclitaxel-freisetzender Ballonkatheter, dessen Ballon teilweise oder vollständig beschichtet ist, **dadurch gekennzeichnet, dass** die Beschichtung die Zusammensetzung gemäß Anspruch 1 umfasst, wobei der Hilfsstoff ausgewählt ist aus der Gruppe bestehend aus zwei oder mehreren unterschiedlichen Triglyceriden gemäß Formel 1, wobei für mindestens eines dieser Triglyceride mehr als 50 Gew.-% der Carbonsäuregruppen des mindestens einen Triglycerids insgesamt Caprylsäure ist.

**9.** Paclitaxel-freisetzender Ballonkatheter gemäß Anspruch 8, wobei die Dosis von Paclitaxel in der Beschichtung im Bereich von 1,5 $\mu g/mm^2$ bis 5 $\mu g/mm^2$ liegt.

**10.** Verfahren zur Herstellung eines Paclitaxel-freisetzender Ballonkatheters, umfassend die folgenden Schritte:

Bereitstellen eines Ballonkatheters; und
Beschichten des Ballonkatheters teilweise oder vollständig mit einer Zusammensetzung gemäß Anspruch 1.

**11.** Verfahren gemäß Anspruch 10, wobei das Beschichten des Ballons die Schritte umfasst:

Bereitstellen einer Formulierung der Zusammensetzung gemäß Anspruch 1 in einem Lösungsmittel;
Sprühen der Formulierung mindestens einmal über den Ballon, um dessen äußere Oberfläche teilweise oder vollständig zu beschichten; und
Trocknen der Beschichtung.

**12.** Verfahren gemäß Anspruch 11, wobei die Formulierung eine Konzentration zwischen 1 bis 10 % nichtflüchtiger Bestandteile aufweist.

**13.** Verfahren gemäß Anspruch 11, wobei das Lösungsmittel ein polares organisches Lösungsmittel oder ein wasserlösliches organisches Lösungsmittel ist, das Wasser in einem Volumenverhältnis von Lösungsmittel/Wasser von 99/1 bis 80/20 enthält, wobei das Lösungsmittel ausgewählt ist aus der Gruppe umfassend trockenes Ethanol, trockenes Methanol, trockenes Isopropanol, Ethanol/Wasser 96/4 (w/w), THF/Wasser 9/1 (v/v) oder Aceton/Wasser 9/1 (v/v).

**14.** Verfahren gemäß Anspruch 11, wobei die Formulierung mindestens zwei-, mindestens drei- oder mehr als dreimal über den Ballon gesprüht wird, um eine Mehrschichtbeschichtung zu erhalten.

**15.** Verwendung der Zusammensetzung gemäß Anspruch 1 zur Vorbereitung einer Vorrichtung zur Behandlung der koronaren oder peripheren Angioplastie.

## Revendications

1. Composition de revêtement de cathéter à ballonnet comprenant du paclitaxel et un excipient choisi dans le groupe comprenant au moins un triglycéride selon la formule 1, au moins un composé selon la formule 2 ou un mélange de ceux-ci, dans laquelle le composé de formule 1 est comme suit :

(Formule 1)

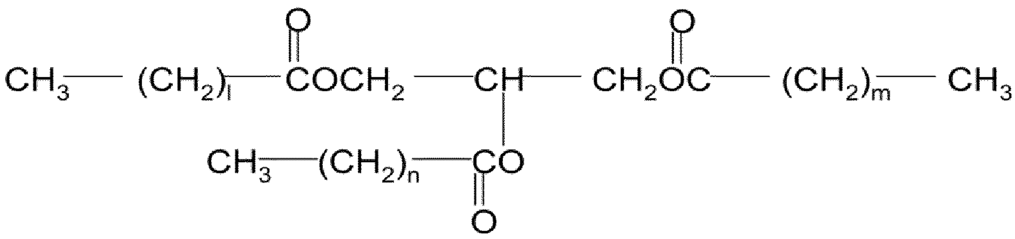

dans laquelle l, m et n sont indépendamment un entier de 4 à 12 ; et
dans laquelle le composé de formule 2 est comme suit :

(Formule 2)

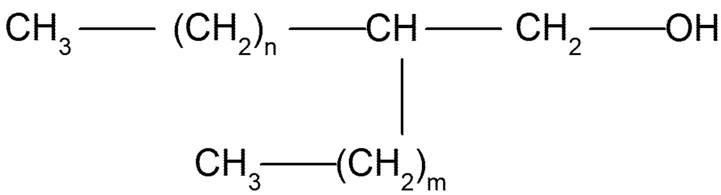

dans laquelle n est un entier de 5 à 11 et m est un entier de 5 à 7 ; et
dans laquelle le rapport pondéral paclitaxel/excipient est compris entre 60/40 et 90/10.

2. Composition selon la revendication 1, dans laquelle le paclitaxel est présent sous forme amorphe et cristalline dans laquelle le rapport pondéral de forme amorphe/cristalline est de 10/90 à 50/50.

3. Composition selon la revendication 1, dans laquelle l'excipient est un liquide à une température de 20 à 25 °C.

4. Composition selon la revendication 1, dans laquelle le composé selon la formule 1 est un triglycéride mixte avec trois acides carboniques différents, de deux acides carboniques différents ou un triglycéride dans lequel l = m = n.

5. Composition selon la revendication 4, dans laquelle le triglycéride selon la formule 1 est le triglycéride d'acide caprylique avec l = m = n = 6.

6. Composition selon la revendication 1,
dans laquelle le composé selon la formule 2 est un composé dans lequel n = 9 et m = 5a9;
ou dans laquelle le composé selon la formule 2 est un composé dans lequel n = 5 a11 etm = 7 ;
ou dans laquelle le composé selon la formule 2 est le 2-octyldodécanol.

7. Composition selon la revendication 1, dans laquelle la composition comprend du paclitaxel, un triglycéride selon la formule 1 et un composé selon la formule 2, dans laquelle le rapport pondéral triglycéride selon la formule 1/composé selon la formule 2 est de 1/99 à 99/1.

8. Cathéter à ballonnet à élution de paclitaxel, dont le ballonnet est partiellement ou complètement revêtu, **caractérisé en ce que** le revêtement comprend la composition selon la revendication 1, dans lequel l'excipient est choisi dans le groupe comprenant deux ou plusieurs triglycérides différents selon la formule 1, dans lequel pour au moins l'un de ces triglycérides, plus de 50 % en poids des groupes acide carbonique du au moins un triglycéride au total sont de l'acide caprylique.

**9.** Cathéter à ballonnet à élution de paclitaxel selon la revendication 8, dans lequel la dose de paclitaxel dans le revêtement va de 1,5 $\mu g/mm^2$ à 5 $\mu g/mm^2$.

**10.** Procédé de fabrication d'un cathéter à ballonnet à élution de paclitaxel comprenant les étapes suivantes :

fourniture d'un cathéter à ballonnet ; et
revêtement partiel ou complet du cathéter à ballonnet avec une composition selon la revendication 1.

**11.** Procédé selon la revendication 10, dans lequel le revêtement du ballonnet comprend les étapes de :

fourniture d'une formulation de la composition selon la revendication 1 dans un solvant ;
pulvérisation de la formulation au moins une fois sur le ballonnet afin de revêtir partiellement ou complètement sa surface externe ; et
séchage du revêtement.

**12.** Procédé selon la revendication 11, dans lequel la formulation a une concentration comprise entre 1 et 10 % de matières non volatiles.

**13.** Procédé selon la revendication 11, dans lequel le solvant est un solvant organique polaire ou un solvant organique hydrosoluble contenant de l'eau dans un rapport volumique solvant/eau de 99/1 à 80/20, dans lequel le solvant est choisi dans le groupe comprenant éthanol sec, méthanol sec, isopropanol sec, éthanol/eau 96/4 (p/p), THF/eau 9/1 (v/v) ou acétone/eau 9/1 (v/v).

**14.** Procédé selon la revendication 11, dans lequel la formulation est pulvérisée au moins deux, au moins trois ou plus de trois fois sur le ballonnet pour obtenir un revêtement multicouche.

**15.** Utilisation de la composition selon la revendication 1 pour la préparation d'un dispositif pour le traitement de l'angioplastie coronaire ou périphérique.

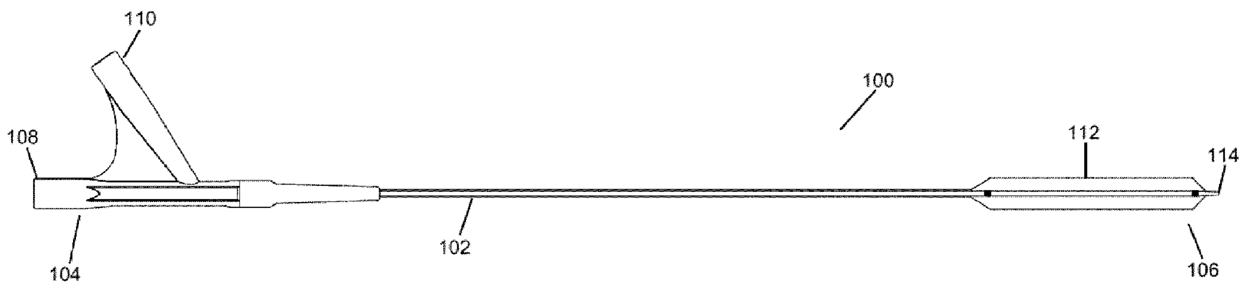


Fig. 1

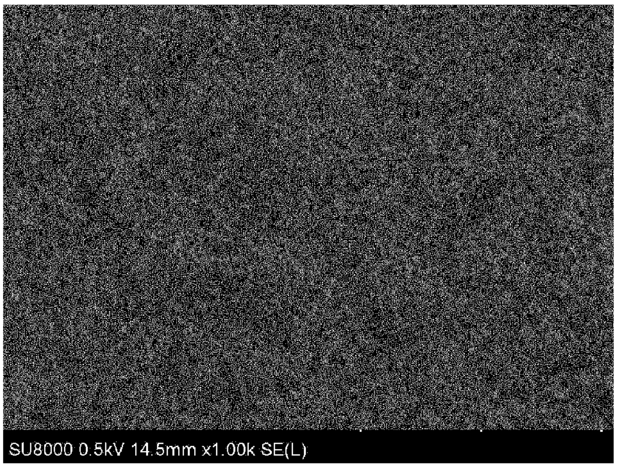


Fig. 2A

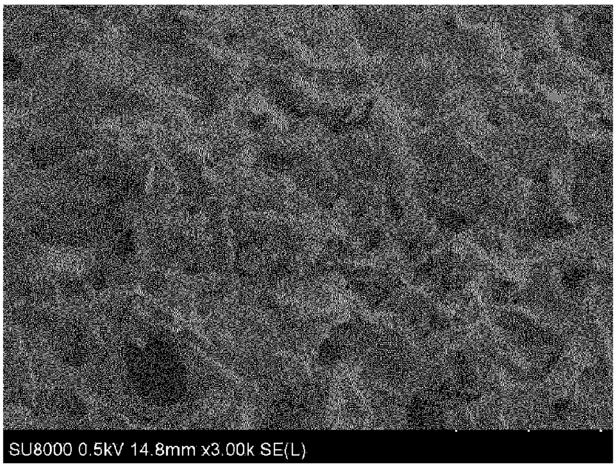


FIG. 2B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010029105 A2